# EUROPÄISCHE PATENTSCHRIFT

(11) **EP 3 484 386 B1**
(45) Veröffentlichungstag und Bekanntmachung des Hinweises auf die Patenterteilung: **30.09.2020**
(21) Anmeldenummer: 18712078.7
(22) Anmeldetag: 06.03.2018
(51) Int. Cl.: A61B 17/34, A61B 17/02, A61B 1/00

(54) **VORRICHTUNG ZUM ZUGANG IN DAS INNERE EINES KÖRPERS**
DEVICE FOR ACCESSING THE INSIDE OF A BODY
DISPOSITIF POUR ACCÉDER À L'INTÉRIEUR D'UN CORPS

(30) Priorität: 16.03.2017 DE 102017002527
(43) Veröffentlichungstag der Anmeldung: 22.05.2019
(73) Patentinhaber: Joimax GmbH, 76227 Karlsruhe (DE)
(72) Erfinder: GIBSON, Alastair, Edinburgh EH10 7JQ (GB); STEEGMÜLLER, Rainer, 70839 Gerlingen (DE); RIES, Wolfgang, 76351 Linkenheim-Hochstetten (DE)
(74) Vertreter: Lichti - Patentanwälte Partnerschaft mbB
(86) Internationale Anmeldenummer: PCT/EP2018/000089
(87) Internationale Veröffentlichungsnummer: WO 2018/166649

(56) Entgegenhaltungen:
- US-A- 5 345 927
- US-A- 5 782 800
- US-A1- 2006 041 270
- US-A1- 2008 058 591
- US-A1- 2010 240 959
- US-A1- 2013 197 592
- US-A1- 2014 025 045

## Beschreibung

Die Erfindung betrifft eine Vorrichtung zum Zugang in das Innere eines Körpers nach dem Oberbegriff des Anspruchs 1.

(Außen-)Rohre oder Hülsen einer derartigen Vorrichtung werden von der Oberfläche eines menschlichen oder tierischen Körpers in das Innere hineingeführt mittels bekannter Techniken, wie insbesondere über einen Einführungskanal schaffenden und erweiternden Dilatoren, wie dies beispielsweise unter anderem in der DE 10 2013 004 964 A1 beschrieben ist. Durch das so gelegte Rohr oder die Hülse kann dann ein Endoskop oder können Arbeitsinstrumente eingeführt werden, um im distalen Endbereich des Rohres bzw. jenseits desselben chirurgische Tätigkeiten zu entfalten, beispielsweise in einem Wirbelzwischenraum Teile einer einen Nerv der Wirbelsäule einklemmenden Bandscheibe zu entfernen, in Wirbel Stabilisierungsmaterial für diese einzubringen oder dergleichen.

Nachteilig ist, dass der Blick- und Arbeitsbereich am distalen Ende des Rohres durch den Rohrdurchmesser selbst begrenzt ist. Insbesondere muss der Rohrdurchmesser ein Kompromiss sein, da man an der Durchführungsstelle durch die Haut eines Patienten nicht mit zu großem Durchmesser von Rohren hindurchtreten möchte, um Beeinträchtigungen möglichst zu minimieren und Narbenbildung zu reduzieren.

Die US 2013/0197592 A1 zeigt eine Verankerungskanüle mit einem Rohr, dessen Ende mit einem Spreizteil versehen ist, das vom distalen Ende in distaler Richtung verlaufende Streifen aufweist, die durch ein "shapeset"-Material miteinander verbunden sind. Innerhalb eines äußeren Rohres werden die Streifen durch dieses in zueinander paralleler und achsparalleler Ausrichtung gehalten, während sie beim Austreten aus einem solchen äußeren Rohr sich konisch aufstellen. Das Spreizteil besteht vorzugsweise aus Polymer. Das Teil wird durch gerade Streifen gebildet, die durch Verbindungsmaterial verbunden sind, so dass ein im aufgestellten Zustand umfangsmäßig geschlossener Trichter gebildet wird. Dies ist nachteilig, da hierdurch Flüssigkeitsfluss behindert wird. Die gerade konische Ausbildung birgt die Gefahr von Verletzungen.

Der Erfindung liegt die Aufgabe zugrunde, unter Beibehaltung der letztgenannten Vorgabe, insbesondere im Hautbereich Einwirkungen so gering wie möglich zu halten, dennoch im Inneren des Körpers, am Arbeitsort für den Chirurgen, ein möglichst optimales und großes Sicht- und Arbeitsfeld unter Vermeidung der Gefahr von Verletzungen zu schaffen.

Erfindungsgemäß wird die genannte Aufgabe mit einer Vorrichtung der eingangs genannten Art gelöst, die die kennzeichnenden Merkmale des Anspruchs 1 aufweist.

Durch das Spreizteil des Innenrohres, das sich beim Austreten aus dem Außenrohr über dessen Durchmesser hinaus aufweitet, wird umgebendes (Weich-)Gewebe zur Seite gedrängt und damit ein Sicht- und Arbeitsbereich mit einem größeren Durchmesser geschaffen, als dies bisher der Fall ist bzw. war. Damit wird das Arbeiten des Operateurs erleichtert und für den Patienten weniger belastend und auch sicherer.

In bevorzugter Weiterbildung ist vorgesehen, dass das Spreizteil als Gitter ausgebildet ist, wobei insbesondere das Spreizteil als Gitter mit rautenförmigen Durchbrechungen ausgebildet ist. Durch diese konstruktive Ausgestaltung des Spreizteils können biokompatible Materialien eingesetzt werden, wie insbesondere Nitinol für zumindest das Spreizteil oder aber auch Edelstahl und es können andere problematischere Materialien, wie sie beispielsweise bei einer schirmartigen Aufweitung oder Durchbrechungen des Spreizteils bei anderen Materialien gegeben wären, vermieden werden.

Eine bevorzugte Weiterbildung sieht dabei vor, dass der Durchmesser des Spreizteils in dessen aufgespreizter Konfiguration sich von einem Übergangsbereich zum Innenrohr hin in distaler Richtung konisch erweiternd erstreckt. Derart ist das Spreizteil in seiner expandierten Konfiguration an die sonstigen Arbeits-Elemente und -Umgebung angepasst, indem sein distales Ende die größte Weite aufweist, während der proximale Übergang des Spreizteils zum distalen Ende der von Innen- und Außenrohr eher stetig ist.

Eine weitere äußerst bevorzugte Ausgestaltung sieht vor, dass das Spreizteil bei aufgespreizter Konfiguration in seinem distalen Bereich zylinderförmig ausgebildet ist. Somit wird eine Verletzungsgefahr reduziert.

In konkreter Ausgestaltung ist dabei vorgesehen, dass das Spreizteil durch miteinander verbundene Streben und zwischen diesen liegenden Schlitzen ausgebildet ist, wobei insbesondere eine Strebe mit in Umfangsrichtung neben ihr verlaufenden unmittelbar benachbarten Streben über eine Verbindungsstelle verbunden ist.

Weiterbildungen der erfindungsgemäßen Vorrichtung sehen vor, dass zwei in Umfangsrichtung nebeneinander angeordnete Streben mit sich ihnen axial unmittelbar anschließenden Streben über eine Verbindungsstelle verbunden sind, wobei insbesondere bei aufgespreiztem Spreizteil vier Streben einen rautenförmigen Zwischenraum umschließen.

Es kann dabei vorgesehen sein, dass einen in aufgespreizter Konfiguration rautenförmigen Zwischenraum umgebende aufeinanderfolgende Streben unterschiedliche Länge haben, wobei insbesondere aufeinanderfolgende Streben unterschiedlicher Länge im proximalen Bereich des Spreizteils ausgebildet sind. In anderen Bereichen des Spreizteils kann vorgesehen sein, dass in aufgespreizter Konfiguration einen rautenförmigen Zwischenraum begrenzende axial aufeinanderfolgende Streben gleiche Länge aufweisen, wobei insbesondere aufeinanderfolgende Streben im distalen Bereich des Spreizteils gleiche Länge aufweisen.

Die Verletzungsgefahr umgebenden Gewebes wird weiterhin dadurch reduziert, dass in bevorzugter Weise vorgesehen ist, dass freie distale Enden der Streben abgerundet sind.

Das Material zumindest des Spreizteils des Innenrohrs bzw. Innenteils besteht äußerst vorzugsweise aus Nitinol, wenn es auch grundsätzlich aus anderen biokompatiblen Materialien, wie beispielsweise Edelstahl, bestehen kann. Nitinol weist dabei den Vorteil auf, dass es zwei temperaturabhängige stabile Zustände hat. So kann das Spreizteil in einer Niedertemperaturkonfiguration bei tiefen Temperaturen eine komprimierte durchgängig zylindrische Konfiguration zum Einführen in und durch das Außenrohr (außerhalb des Körpers eines Patienten) und beim Einführen mit dem Außenrohr in den Körper des Patienten aufweisen, während das Spreizteil sich dann erst beim Ausfahren aus dem distalen Ende des Außenrohrs expandiert und zu seiner im Wesentlichen expandierten Konfiguration aufweitet, bei der, wie gesagt, vorzugsweise der äußerste distale Bereich wiederum zylindrisch ausgebildet ist.

Um eine sichere Verbindung des Spreizteils mit einem üblicherweise aus Edelstahl bestehenden Innenrohr zu schaffen, wird, da die beiden Materialien schlecht stoffschlüssig verbindbar sind, insbesondere nur eingeschränkt verschweißbar sind, vorgesehen, dass das Spreizteil einstückig mit einem ebenfalls aus Nitinol bestehenden distalen Rohrabschnitt des inneren Rohres ausgebildet ist. Zur Durchführung der formschlüssigen Verbindung ist insbesondere vorgesehen, dass der distale Rohrabschnitt in seinem proximalen Bereich bis zu seiner proximalen Stirnseite ragende Schlitze aufweist, die sich insbesondere von den radialen Vorsprüngen des proximalen Rohrabschnitts umgehenden Ausnehmungen aus erstrecken.

Eine weitere bevorzugte Ausgestaltung sieht vor, dass Außen- und Innenrohr an ihren proximalen Enden miteinander zusammenwirkende Griffteile aufweisen, die bei aufgespreizter Konfiguration des Spreizteils einen gemeinsamen Griff bilden, wobei insbesondere ein distaler Abschnitt des Griffteils des Innenteils ein Außengewinde aufweist, das mit einem in dieses eingreifenden nach innen ragenden Stift des Griffteils des Außenrohres zusammenwirkt. Hierdurch kann in definierter gesteuerter und gezielter Weise das Spreizteil des Außenrohres herausgeführt werden, wobei dieses sicherlich vorzugsweise zurückbewegt, indem Innen- und Außenteil relativ zueinander gedreht werden und damit durch die schraubenförmige Verbindung auch eine axiale Relativbewegung der beiden Teile bewirkt wird.

Zur Verbindung von Außen- und/oder Innenrohr mit dem jeweiligen Griffteil ist in weiterer bevorzugter Ausgestaltung vorgesehen, dass das Außenrohr mit einem ersten Griffteil eines Griffs und/oder das Innenrohr mit einem zweiten Griffteil eines Griffs formschlüssig verbunden sind, wobei insbesondere das Außenrohr und/oder das Innenrohr jeweils an ihrem proximalen Ende nach außen gerichtete Nasen mit Hinterschneidungen aufweist/aufweisen, die mit auf der Innenseite des jeweiligen Griffteils ausgebildete Hinterschneidungen hintergreifen.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung erläutert ist. Dabei zeigt:
- Fig. 1: Eine erfindungsgemäße Vorrichtung in Arbeitskonfiguration in Seitenansicht;
- Fig. 2: Vorrichtung der Fig. 1 in einer perspektivischen Darstellung mit Schrägsicht auf das aufgespreizte distale Ende;
- Fig. 3: einen Längsschnitt durch eine erfindungsgemäße Vorrichtung in Einführkonfiguration derselben;
- Fig. 3a: eine vergrößerte Ausschnittdarstellung des distalen Endes der Fig. 3;
- Fig. 4: einen Längsschnitt entsprechend Fig. 2 der Vorrichtung in Arbeitskonfiguration entsprechend der Fig. 1;
- Fig. 5: einen Längsschnitt durch das Innenteil der erfindungsgemäßen Vorrichtung in gegenüber der Fig. 4 etwas vergrößerter Darstellung;
- Fig. 6: einen Längsschnitt durch das distale Ende des Innenteils der erfindungsgemäßen Vorrichtung;
- Fig. 7: eine Seitenansicht des Innenteils der erfindungsgemäßen Vorrichtung entsprechend der Fig. 5;
- Fig. 8: einen Längsschnitt des Innenrohrs der erfindungsgemäßen Vorrichtung entsprechend E-E der Fig. 7;
- Fig. 9: eine vergrößerte Längsschnitt-Ausschnittdarstellung des proximalen Bereichs des Innenteils der erfindungsgemäßen Vorrichtung; und
- Fig. 10: einen Längsschnitt entsprechend Fig. 9 des proximalen Bereichs des Außenteils der erfindungsgemäßen Vorrichtung.

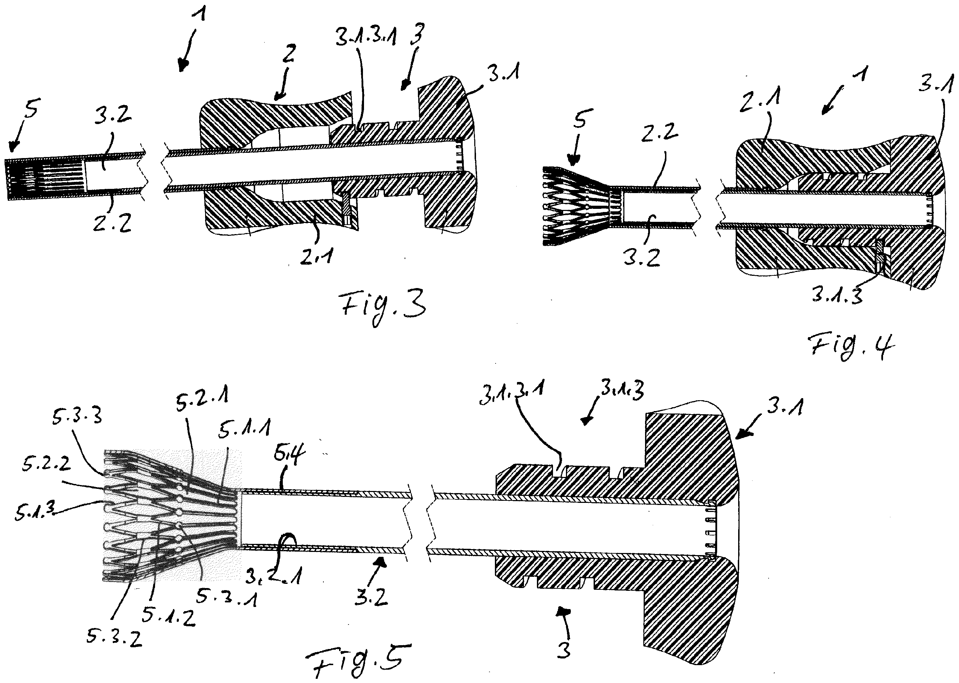

Die erfindungsgemäße Vorrichtung 1 zum Zugang in einen Körper weist ein Außenteil 2 und ein Innenteil 3 (insbesondere Fig. 3, 5) auf.

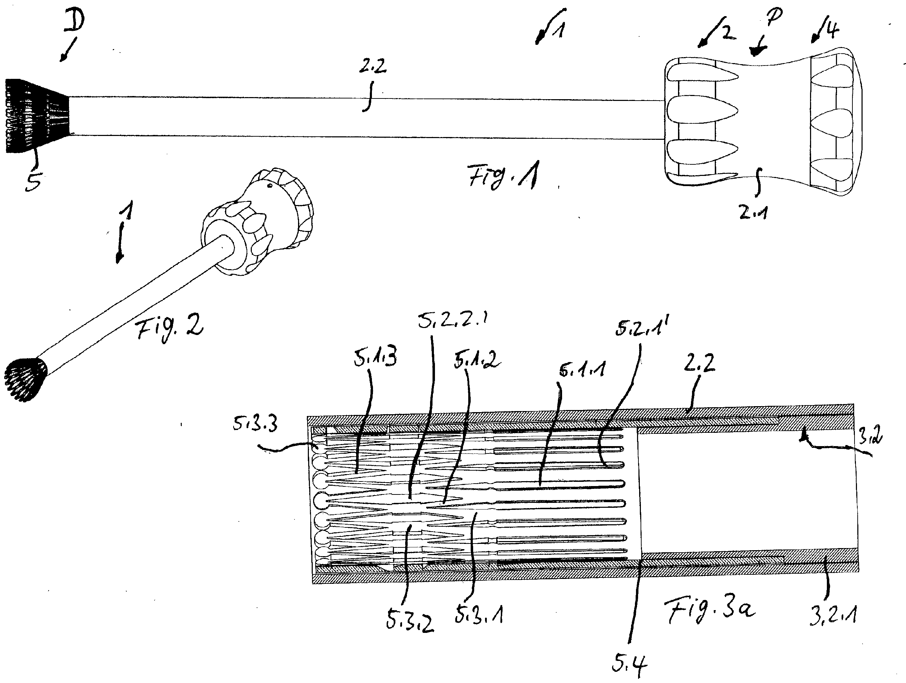

Das Außenteil 2 weist einen proximalen Endbereich P der Vorrichtung, ein erstes Griffteil 2.1 und weiterhin ein Außenrohr 2.2 auf. Das Innenteil 3 weist zunächst ein zweites Griffteil 3.1 und weiterhin ein in das Außenrohr 2.2 ein- und durch dieses hindurchführbares Innenrohr 3.2 auf. Erstes Griffteil 2.1 und zweites Griffteil 3.1 bilden gemeinsam beispielsweise in in den Fig. 1 und 2 dargestellten Arbeitskonfigurationen einen einheitlichen Griff 4.

Das Innenteil 3 weist an seinem distalen Ende D ein Spreizteil 5 auf. Dieses besteht aus Nitinol und nimmt zumindest bei über der Umgebungstemperatur - von ca. 20C° bis 22C° - liegenden Übergangstemperatur von mindestens 25C°, vorzugsweise 28°C, eine aufgespreizte Spreizkonfiguration dar, wie sie für das Spreizteil in sämtlichen diesen darstellenden Figuren, außer der Fig. 3a, ersichtlich ist.

Vorzugweise weist das Spreizteil 5 bei tieferen Temperaturen unterhalb der Übergangstemperatur eine selbstständige kontrahierte Konfiguration auf, wie sie der innerhalb des Außenrohres der Darstellung der Fig. 3, 3a entsprechen. Die Ausbildung von Nitinol ist die bevorzugte Ausgestaltung. Alternativ kann auch vorgesehen sein, dass das Spreizteil 5 aus gleichem Material oder einem anderen Material, wie Edelstahl, mit einer stabilen Spreizkonfiguration entsprechend beispielsweise der Fig. 4 durch ein konisch nach innen verlaufendes Einführelement vom distalen Ende des Außenteils 2 bzw. dessen Außenrohres 2.2 zwangsweise radial komprimiert und derart in das Außenrohr 2.2 eingeführt wird.

Das Spreizteil 5 ist als Gitter in Form einer Tulpe ausgebildet. Es weist einzelne Streben 5.1.1, 5.1.2, 5.1.3 auf, zwischen denen in der komprimierten oder Tieftemperatur-Konfiguration (Fig. 3a) Schlitze 5.2.1', 5.2.2' ausgebildet sind, die sich in der expandierten oder HochtemperaturKonfiguration zu Zwischenräumen zwischen den Streben 5.1.1, 5.1.2 in Form von Rauten 5.2.1, 5.2.2 aufweiten (beispielsweise Fig. 5). Die Rauten 5.2.1, 5.2.2 können dabei symmetrisch (5.2.2) oder asymmetrisch (5.2.1) ausgebildet sein - und zwar relativ zu einer Radialebene des Spreizteils durch ihre jeweiligen Bereiche größerer Breite in Längsrichtung. Die einen Zwischenraum in Form einer Raute 5.2.1, 5.2.2 umgebende Streben 5.1.2, 5.1.3 die gleiche Länge haben oder aber jeweils zwei Streben 5.1.1, 5.1.2 unterschiedliche Länge haben; so sind die Streben 5.1.1 länger als die Streben 5.1.2, 5.1.3 der gleichen Rauten.

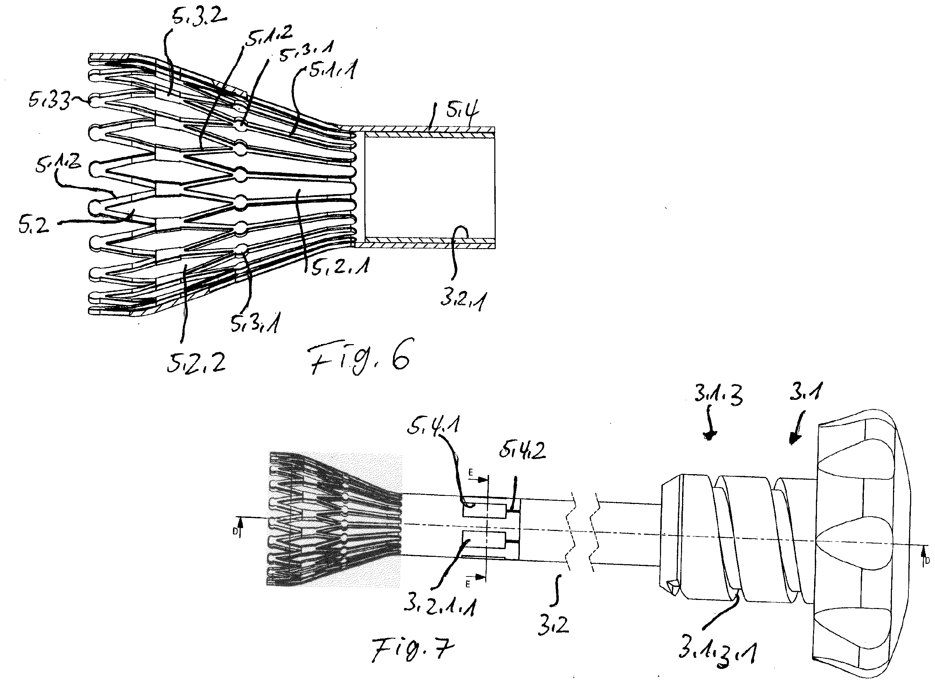

Eine Strebe 5.1.1, 5.1.2, 5.1.3 ist ein gestreckter Metallabschnitt des Spreizteils 5 von einer Verbindungsstelle 5.3.1 mit einer anderen Strebe bis zu einer anderen Verbindungsstelle mit einer dritten Strebe (insbesondere Fig. 6). Insbesondere aus der Fig. 6 ist ersichtlich, dass die den dortige Zwischenraum-Raute 5.2.1 mitbildenden proximalen Streben 5.1.1 länger ausgebildet sind, als die auf den Streben 5.1.1 distal folgenden die gleiche Raute 5.2.1 mitbildenden Streben 5.1.2, während die distal auf die Raute 5.2.1 folgende Zwischenraum-Raute 5.2.2 von sie umgebenden Streben 5.1.2, 5.1.3 gleicher Länge gebildet ist. Die proximale Verbindungsstelle 5.3.1 verbindet drei Streben und zwar eine Strebe 5.1.1 mit zwei Streben 5.1.2. Die mittlere Verbindungsstelle 5.3.2 verbindet vier Streben 5.1.2, 5.1.3 gleicher Länge und die am distalen Ende des Spreizteils 5 ausgebildete Verbindungsstelle 5.3.3 verbindet zwei Streben 5.1.3.

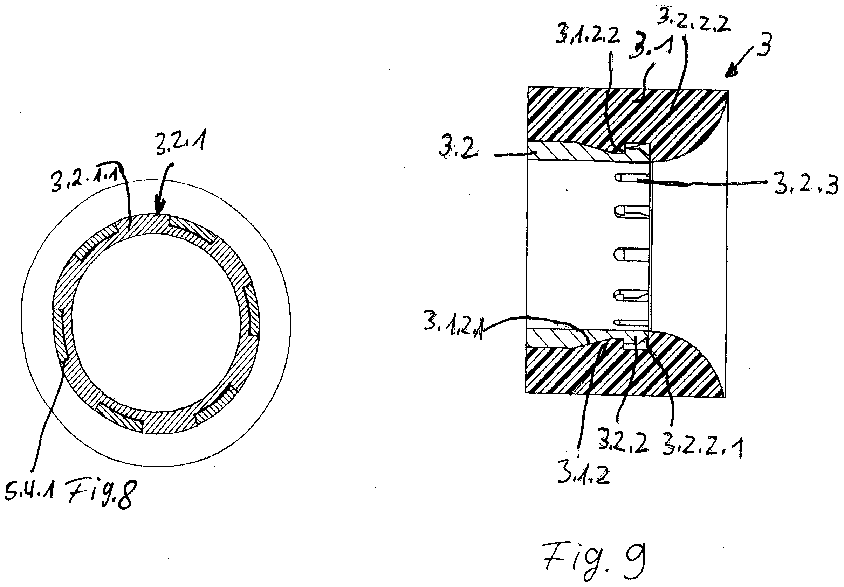

Das Spreizteil 5 ist einstückig mit einem distalen Rohrabschnitt 5.4 des Innenrohrs 3 ausgebildet. Der Rohrabschnitt 5.4 ist einstückig mit dem Spreizteil 5 ausgebildet und besteht bevorzugterweise ebenfalls aus Nitinol, während der einstückig am Innenrohr 3.2 ausgebildete Rohrabschnitt 3.2.1 ebenso wie dieses aus Edelstahl besteht. Der Rohrabschnitt 5.4 überlappt einen distalen Endabschnitt 3.2.1, wobei der Abschnitt 5.4 den Abschnitt 3.2.1 radial außenseitig umgibt. Rohrabschnitt 5.4 und Rohrabschnitt 3.2.1 sind formschlüssig miteinander verbunden, wie dies in den Fig. 7 und 8 dargestellt ist, da stoffschlüssige Verbindungen schlecht machbar sind, insbesondere eine Schweißverbindung zwischen den Materialien Nitinol und Edelstahl nicht möglich ist und Klebverbindungen aus organischen Stoffen bei in einen menschlichen Körper eingebrachten Elementen nicht zulässig sind und darüber hinaus nicht zuverlässig sind, da hier eine Trennung nicht ausgeschlossen ist.

Die formschlüssige Verbindung ist derart ausgestaltet, dass der Rohrabschnitt 3.2.1 des Innenrohrs 3.1 an seiner Außenseite einstückig ausgebildete rechteckige Vorsprünge 3.2.1.1 aufweist, während der Rohrabschnitt 5.4 an dieses angepasste rechteckige Ausnehmungen 5.4.1 hat und mit diesem die Vorsprünge 3.2.1.1 übergreift, wie dies insbesondere der Fig. 7 ersichtlich ist. Damit der Rohrabschnitt 5.4 über den Rohrabschnitt 3.2.1 geschoben werden kann, sind proximal zu den Ausnehmungen 5.4.1 am Rohrabschnitt 5.4 Schlitze 5.4.2 ausgebildet (Fig. 7).

Hierdurch ist eine zuverlässige formschlüssige Verbindung der beiden Rohrabschnitte 5.4 und 3.2.1 erzielbar. Die Bildung einerseits der Vorsprünge 3.2.1.1, andererseits der Ausnehmungen 5.4.1 erfolgt vorzugsweise in spanabhebender Weise, beispielsweise durch Laserschneiden, Fräsen, Schleifen oder auch hinsichtlich der Vorsprünge 3.2.1.1 Wegätzen von Bereichen des Rohrabschnitts 3.2.1 in die Vorsprünge 3.2.1.1 umgebenden Bereichen.

Das Innenrohr 3.2 ist an seinem proximalen Ende ebenfalls formschlüssig mit dem Griffteil 3.1 verbunden, wie dies in der Fig. 9 dargestellt ist.

Hierzu weist das Innenrohr 3.2 an seinem proximalen Ende sich radial erstreckende Nasen 3.2.2 auf, die durch eine unmittelbar distal der Nasen 3.2.2 vorgesehe Verjüngung der Wandung des Innenrohres 3.2 gebildet sind. Die Verjüngung kann ebenfalls wieder in der beschriebenen Weise spanabhebend oder durch Ätzen geschaffen sein. Damit die Nasen 3.2.2 radial nach innen zurückweichen können, sind zwischen ihnen Schlitze 3.2.3 ausgebildet. Die Nasen hintergreifen einen nach innen ragenden umlaufenden Vorsprung 3.1.2 des Griffteils 3.1. Damit sie aus distaler Richtung zur Herstellung der formschlüssigen Verbindung an diesem Vorsprung 3.1.2 vorbeigleiten können, weist dieser auf seiner distalen Seite 3.1.2.1 ebenso eine Abschrägung auf, wie die Nasen 3.2.2 an ihrem proximalen Ende 3.2.2.1, während die proximale Stirnseite 3.1.2.2 der Nase 3.2.2 und die distale Seite 3.2.2.2 der Nase 3.2.2 jeweils radial gerichtet sind.

Hierdurch lässt sich eine sichere und zuverlässige formschlüssige Verbindung von Innenrohr 3.2 und Griffteil 3.1 des Innenteils 3 schaffen.

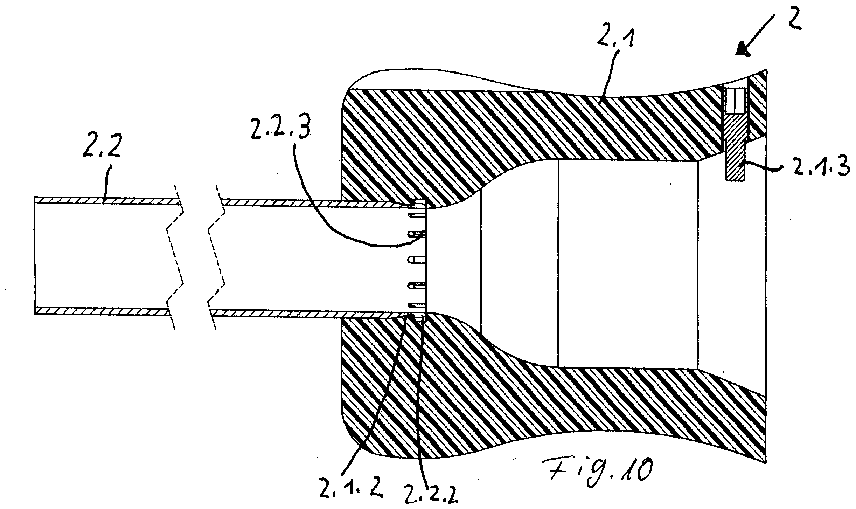

Die Fig. 10 zeigt, dass das Außenrohr 2.2 mit seinem Griffteil 2.1 in der gleichen Weise formschlüssig verbunden ist.

Hierzu weist das Außenrohr 2.2 ebenso wie das Innenrohr 3.2 an seinem proximalen Ende sich radial erstreckende Nasen 2.2.2 auf, die durch eine Verjüngung der Wandung des Innenrohres 2.2 unmittelbar distal der Nasen 2.2.2 gebildet sind. Die Verjüngung kann ebenfalls spanabhebend oder durch Ätzen gebildet sein. Damit die Nasen 2.2.2 radial nach innen zurückweichen können, sind zwischen ihnen Schlitze 2.2.3 ausgebildet. Die Nasen 2.2.2 hintergreifen einen nach innen ragenden umlaufenden Vorsprung 2.1.2 des Griffteils 2.1. Damit sie aus distaler Richtung zur Herstellung der formschlüssigen Verbindung an diesem Vorsprung 2.1.2 vorbeigleiten können, weist dieser auf seiner distalen Seite ebenso eine Abschrägung auf, wie die Nasen 2.2.2 an ihrem proximalen Ende, während die proximale Stirnseite der Nase 2.2.2 und die distale Seite der Nase 2.2.2 jeweils radial gerichtet sind.

Hierdurch lässt sich eine sichere und zuverlässige formschlüssige Verbindung von Außenrohr 2.2 und Griffteil 2.1 des Außenteils 2 schaffen.

Außenteil 2 und Innenteil 3 sind in folgender Weise zueinander bewegbar miteinander verbunden. Das heißt durch eine relative Drehbewegung der Teile zueinander erfolgt auch eine relative Axialbewegung derselben. Hierzu weist (insbesondere Fig. 5, 7) das Griffteil 3.1 des Innenteils 3 über einen distalen Abschnitt 3.1.3 ein äußeres Schraubgewinde 3.1.3.1 auf, in welches ein nach innen ragender Stift 2.1.3 am proximalen Ende des Griffteils 2.1 des Außenteils 2 eingreift, wie dies neben den Fig. 5, 7, 10 insbesondere dem Vergleich der Fig. 3 und 4 entnehmbar ist.

Der Einsatz der erfindungsgemäßen Vorrichtung 1 geschieht folgendermaßen:
Zunächst wird das Innenrohr 3.2 des Innenteils 3, wenn das Spreizteil 5 aus Nitinol besteht, bei relativ tiefer Temperatur, indem das Spreizteil 5 seine in der Fig. 3 dargestellte komprimierte Stellung einnimmt, in das Außenrohr 2.2 des Außenteils 2 bis zu der Position der Fig. 3 eingeführt, also ohne dass das Spreizteil 5 aus dem distalen Ende des Außenrohrs 2.2 hervorragt. Dabei greift der Stift 2.1.3 am distalen Ende des Schraubgewindes 3.1.3.1 in dieses ein. Anschließend wird die Vorrichtung in dieser Konfiguration bis zum Arbeitsort im Inneren eines Körpers, wie eines menschlichen oder tierischen Körpers, eingeführt. Anschließend werden Innen- und Außenteil relativ zueinander verdreht, wodurch sich das Spreizteil 5 aus dem distalen Ende des Außenrohrs 2.2 herausbewegt, wie dies in der Fig. 4 dargestellt ist, bis er seine dort dargestellte aufgespreizte oder Hochtemperaturkonfiguration einnimmt.

Die Länge des Spreizteils weist vorzugsweise 8 mm bis 28 mm, insbesondere 18 mm auf, während der Durchmesser im Bereich von 5 mm bis 30 mm, insbesondere bei 17 bis 18 mm liegt. Insbesondere liegt das Verhältnis von Länge zu Durchmesser des Spreizteils 5 zwischen 0,25 und 1, vorzugsweise bei 0,7 bis 0,8. Darüber hinaus liegt der Durchmesser des distalen Endes des Spreizteils 5 in der expandierten oder Hochtemperaturstellung von dem 1,5- bis 2,5-fachen des Durchmessers der Rohre 2.2, 2.3, vorzugsweise beim Doppelten.

Die Funktion des Spreizteils 5 ist, das es umgebene Körpergewebe zur Seite zu drücken und damit einen radial größeren freien Bereich zu schaffen, zum einen zum Einblick durch ein Endoskop, zum anderen hinsichtlich des Arbeitens mittels Instrumenten, beispielsweise Weichgewebe vor Knochenteilen, wie Wirbeln der Wirbelsäule aufzuweiten, so dass ein größeres Blickfeld auf den Wirbel oder einen Wirbelzwischenraum geschaffen und ein größeres Blick- und Arbeitsfeld auf bzw. an einem Wirbel oder einem Wirbelzwischenraum geschaffen wird. Hierdurch wird das Arbeiten eines Operateurs wesentlich erleichtert.

## Patentansprüche

1. Vorrichtung (1) zum Zugang in das Innere eines Körpers, mit mindestens einem ein Außenrohr (2.1) aufweisenden Außenteil (2), wobei ein durch das Außenrohr hindurchführendes Innenrohr (3.2) an seinem distalen Ende (D) einen über den Außendurchmesser des Außenrohrs hinaus aufweitbaren Spreizteil (5) aufweist, **dadurch gekennzeichnet, dass** ein distaler Rohrabschnitt (3.2.1) des Innenrohrs (3.2) und ein proximaler Abschnitt (5.4) des Spreizteils (5) derart formschlüssig miteinander verbunden sind, dass der distale Rohrabschnitt (3.2.1) des Innenrohrs (3.2) auf seiner Außenseite radiale Vorsprünge (3.2.1.1) aufweist, die in Ausnehmungen (5.4.1) im proximalen Abschnitt (5.4) des Spreizteils (5) eingreifen und dass das Spreizteil (5) bei aufgespreizter Konfiguration in seinem distalen Bereich zylinderförmig ausgebildet ist.

2. Vorrichtung nach Anspruch 1, **dadurch gekennzeichnet, dass** der proximale Abschnitt (5.4) bis zu seiner proximalen Stirnseite ragende Schlitze (5.4.2) aufweist, die sich insbesondere von radialen Vorsprüngen (3.2.1.1) des proximalen Abschnitts (3.2.1) des Innenrohrs (3.2) umgehenden Ausnehmungen (5.4.1) aus erstrecken.

3. Vorrichtung nach Anspruch 1, **dadurch gekennzeichnet, dass** ein proximaler Abschnitt (5.4) des Spreizteils (5) als Rohrabschnitt ausgebildet ist.

4. Vorrichtung nach einem der vorangehenden Ansprüche, **dadurch gekennzeichnet, dass** das Spreizteil (5) aus Nitinol besteht.

5. Vorrichtung nach einem der vorangehenden Ansprüche, **dadurch gekennzeichnet, dass** ein distaler Rohrabschnitt (3.2.1) des Innenrohrs (3.2) ebenso wie dieses aus Edelstahl besteht.

6. Vorrichtung nach einem der vorangehenden Ansprüche, **dadurch gekennzeichnet, dass** das Spreizteil (5) aus Nitinol besteht und einstückig mit einem ebenfalls aus Nitinol bestehenden distalen Abschnitt (5.4) ausgebildet ist.

7. Vorrichtung nach einem der vorangehenden Ansprüche, **dadurch gekennzeichnet, dass** das Spreizteil (5) als Gitter ausgebildet ist.

8. Vorrichtung nach einem der vorangehenden Ansprüche, **dadurch gekennzeichnet, dass** das Spreizteil (5) im expandierten Zustand als Gitter mit rautenförmigen Durchbrechungen (5.2.1, 5.2.2) ausgebildet ist.

9. Vorrichtung nach einem der vorangehenden Ansprüche, **dadurch gekennzeichnet, dass** der Durchmesser des Spreizteils (5) in dessen aufgespreizter Konfiguration sich von einem Übergangsbereich zum Innenrohr (3.2) hin in distaler Richtung konisch erweiternd erstreckt.

10. Vorrichtung nach einem der vorangehenden Ansprüche, **dadurch gekennzeichnet, dass** das Spreizteil (5) durch miteinander verbundene Streben (5.1.1, 5.1.2) und zwischen diesen liegenden Schlitzen ausgebildet ist, wobei insbesondere eine Strebe (5.1.1, 5.1.2) mit in Umfangsrichtung neben ihr verlaufenden unmittelbar benachbarten Streben (5.1.1, 5.1.2) über eine Verbindungsstelle (5.3.1, 5.3.2, 5.3.3) verbunden ist.

11. Vorrichtung nach einem der vorangehenden Ansprüche, **dadurch gekennzeichnet, dass** zwei in Umfangsrichtung nebeneinander angeordnete Streben (5.1.1, 5.1.2) mit sich ihnen axial unmittelbar anschließenden Streben (5.1.1, 5.1.2) über eine Verbindungsstelle(5.3.1, 5.3.2) verbunden sind.

12. Vorrichtung nach einem der vorangehenden Ansprüche, **dadurch gekennzeichnet, dass** bei aufgespreiztem Spreizteil (5) vier Streben (5.1.1, 5.1.2 bzw. 5.1.2, 5.1.3) einen rautenförmigen Zwischenraum (5.2.1, 5.2.2) umschließen.

13. Vorrichtung nach einem der vorangehenden Ansprüche, **dadurch gekennzeichnet, dass** einen in aufgespreizter Konfiguration rautenförmigen Zwischenraum (5.2.1) umgebende aufeinanderfolgende Streben (5.1.1, 5.1.2) unterschiedliche Länge haben, wobei insbesondere aufeinanderfolgende Streben (5.1.1, 5.1.2) unterschiedlicher Länge im proximalen Bereich des Spreizteils (5) ausgebildet sind.

14. Vorrichtung nach einem der vorangehenden Ansprüche, **dadurch gekennzeichnet, dass** in aufgespreizter Konfiguration einen rautenförmigen Zwischenraum (5.2.2) begrenzende axial aufeinanderfolgende Streben (5.1.2) gleiche Länge aufweisen, wobei insbesondere aufeinanderfolgende Streben (5.1.2) im distalen Bereich des Spreizteils (5) gleiche Länge aufweisen.

15. Vorrichtung nach einem der vorangehenden Ansprüche, **dadurch gekennzeichnet, dass** freie distale Enden der Streben (5.1.2) abgerundet sind.

16. Vorrichtung nach einem der vorangehenden Ansprüche, **dadurch gekennzeichnet, dass** Außen- und Innenrohr (2.2, 3.2) an ihren proximalen Enden miteinander zusammenwirkende Griffteile (2.1, 3.1) aufweisen, die bei aufgespreizter Konfiguration des Spreizteils einen gemeinsamen Griff (4) bilden, wobei insbesondere ein distaler Abschnitt (3.1.3) des Griffteils (3.1) des Innenteils (3) ein Außengewinde aufweist, das mit einem in dieses eingreifenden nach innen ragenden Stift (2.1.3) des Griffteils (2.1) des Außenrohres (2) zusammenwirkt.

17. Vorrichtung nach einem der vorangehenden Ansprüche, **dadurch gekennzeichnet, dass** das Außenrohr (2.2) mit einem ersten Griffteil (2.1) eines Griffs (4) und/oder dass Innenrohr (3) mit einem zweiten Griffteil (3.1) eines Griffs formschlüssig verbunden sind, wobei insbesondere das Außenrohr (2.2) und/oder das Innenrohr (3.2) jeweils an ihrem proximalen Ende nach außen gerichtete Nasen (2.2.2, 3.2.2) mit Hinterschneidungen aufweist/aufweisen die mit auf der Innenseite des jeweiligen Griffteils ausgebildete Hinterschneidungen (2.1.2, 3.1.2) hintergreifen.

## Claims

1. Device (1) for access to the interior of a body, having at least one outer part (2) having an outer tube (2.1), wherein an inner tube (3.2) passing through the outer tube has at its distal end (D) an expanding part (5), which is expandable beyond the outer diameter of the outer tube, **characterized in that** a distal tube section (3.2.1) of the inner tube (3.2) and a proximal section (5.4) of the expanding part (5) are connected to one another in a positive-locking manner so that the distal tube section (3.2.1) of the inner tube (3.2) has on its outer side radial projections (3.2.1.1) that mesh with recesses (5.4.1) in the proximal section (5.4) of the expanding part (5) and that the expanding part (5) has a cylindrical configuration in its distal area in case of an expanded configuration.

2. Device in accordance with claim 1, **characterized in that** the proximal section (5.4) has slots (5.4.2) which protrude up to its proximal end face and which extend especially from recesses (5.4.1) which bypass radial projections (3.2.1.1) of the proximal section (3.2.1) of the inner tube (3.2).

3. Device in accordance with claim 1, **characterized in that** a proximal section (5.4) of the expanding part (5) is configured as a tube section.

4. Device in accordance with one of the above claims, **characterized in that** the expanding part (5) is made of Nitinol.

5. Device in accordance with one of the above claims, **characterized in that** a distal tube section (3.2.1) of the inner tube (3.2) as well as the inner tube are made of stainless steel.

6. Device in accordance with one of the above claims, **characterized in that** the expanding part (5) is made of Nitinol and is configured in one piece with a distal section (5.4) which is likewise made of Nitinol.

7. Device in accordance with one of the above claims, **characterized in that** the expanding part (5) is configured as a grid.

8. Device in accordance with one of the above claims, **characterized in that** in the expanded state, the expanding part (5) is configured as a grid with diamond-shaped openings (5.2.1, 5.2.2).

9. Device in accordance with one of the above claims, **characterized in that** the diameter of the expanding part (5) in the expanded configuration thereof extends conically expanding in the distal direction from a transition area towards the inner tube (3.2).

10. Device in accordance with one of the above claims, **characterized in that** the expanding part (5) is formed by struts (5.1.1, 5.1.2), which are connected to one another, and by slots which are located between the struts (5.1.1, 5.1.2), wherein especially one strut (5.1.1, 5.1.2) is connected via a connection point (5.3.1, 5.3.2, 5.3.3) to struts (5.1.1, 5.1.2) extending directly adjacent to the strut (5.1.1, 5.1.2) in the circumferential direction.

11. Device in accordance with one of the above claims, **characterized in that** two struts (5.1.1, 5.1.2) arranged next to one another in the circumferential direction are connected via a connection point (5.3.1, 5.3.2) to struts (5.1.1, 5.1.2) which are axially directly adjacent to them.

12. Device in accordance with one of the above claims, **characterized in that** four struts (5.1.1, 5.1.2 and 5.1.2, 5.1.3) enclose a diamond-shaped intermediate space (5.2.1, 5.2.2) when the expanding part (5) is expanded.

13. Device in accordance with one of the above claims, **characterized in that** in the expanded configuration struts (5.1.1, 5.1.2) following one another and enclosing a diamond-shaped intermediate space (5.2.1) have different lengths, wherein especially struts (5.1.1, 5.1.2) of different lengths following one another are formed in the proximal area of the expanding part (5).

14. Device in accordance with one of the above claims, **characterized in that** in the expanded configuration struts (5.1.2) axially following one another and enclosing a diamond-shaped intermediate space (5.2.2) have equal lengths, wherein especially struts (5.1.2) following one another have equal lengths in the distal area of the expanding part (5).

15. Device in accordance with one of the above claims, **characterized in that** free distal ends of the struts (5.1.2) are rounded off.

16. Device in accordance with one of the above claims, **characterized in that** the outer tube and the inner tube (2.2, 3.2) have grip parts (2.1, 3.1), which interact with one another at their proximal ends and which form a common grip (4) in case of the expanded configuration of the expanding part, wherein especially a distal section (3.1.3) of the grip part (3.1) of the inner part (3) has an external thread, which interacts with a pin (2.1.3) of the grip part (2.1) of the outer tube (2), which protrudes inwards and meshes with it.

17. Device in accordance with one of the above claims, **characterized in that** the outer tube (2.2) is connected in a positive-locking manner to a first grip part (2.1) of a grip (4) and/or that the inner tube (3) is connected in a positive-locking manner to a second grip part (3.1) of a grip, wherein especially the outer tube (2.2) and/or the inner tube (3.2) each have at their proximal end lugs (2.2.2, 3.2.2) directed outwards with undercuts which engage behind with undercuts formed on the inner side of the respective grip part.

## Revendications

1. Dispositif (1) pour accéder à l'intérieur d'un corps, avec au moins une partie extérieure (2) comportant un tube extérieur (2.1), un tube intérieur (3.2) introduit à travers le tube extérieur au niveau de son extrémité distale (D), une partie d'écartement (5) pouvant s'élargir au-delà du diamètre extérieur du tube extérieur, **caractérisé en ce qu'**une section de tube distale (3.2.1) du tube intérieur (3.2) et une section proximale (5.4) de la partie d'écartement (5) sont reliées de telle sorte entre elles par complémentarité de formes que la section de tube distale (3.2.1) du tube intérieur (3.2) comporte sur son côté extérieur des saillies radiales (3.2.1.1) s'imbriquant dans les évidements (5.4.1) pratiqués dans la section proximale (5.4) de la partie d'écartement (5) et que dans la configuration écartée, la partie d'écartement (5) est réalisée en forme de cylindre dans sa zone distale.

2. Dispositif selon la revendication 1, **caractérisé en ce que** la section proximale (5.4) comporte des fentes (5.4.2) saillant jusqu'à son côté proximal avant, ces fentes s'étendant notamment depuis les évidements (5.4.1) entourant les saillies radiales (3.2.1.1) de la section proximale (3.2.1) du tube intérieur (3.2).

3. Dispositif selon la revendication 1, **caractérisé en ce qu'**une section proximale (5.4) de la partie d'écartement (5) est réalisée sous la forme d'une section de tube.

4. Dispositif selon l'une quelconque des revendications précédentes, **caractérisé en ce que** la partie d'écartement (5) est en nitinol.

5. Dispositif selon l'une quelconque des revendications précédentes, **caractérisé en ce qu'**une section de tube distale (3.2.1) du tube intérieur (3.2) est comme celui-ci en inox.

6. Dispositif selon l'une quelconque des revendications précédentes, **caractérisé en ce que** la partie d'écartement (5) est en nitinol et réalisée d'un seul tenant avec une section distale (5.4) également en nitinol.

7. Dispositif selon l'une quelconque des revendications précédentes, **caractérisé en ce que** la partie d'écartement (5) est réalisée sous la forme d'une grille.

8. Dispositif selon l'une quelconque des revendications précédentes, **caractérisé en ce que** la partie d'écartement (5) est réalisée dans l'état étendu sous la forme d'une grille avec des trous traversants (5.2.1, 5.2.2) en forme de losange.

9. Dispositif selon l'une quelconque des revendications précédentes, **caractérisé en ce que** le diamètre de la partie d'écartement (5) s'étend dans sa configuration écartée en s'élargissant de façon conique depuis une zone de transition menant au tube intérieur (3.2), dans la direction distale.

10. Dispositif selon l'une quelconque des revendications précédentes, **caractérisé en ce que** la partie d'écartement (5) est réalisée par des étais (5.1.1, 5.1.2) reliés entre eux et des fentes disposées entre eux, un étai (5.1.1, 5.1.2) étant notamment relié aux étais (5.1.1, 5.1.2) s'étendant de façon directe connexe à côté de lui dans la direction périphérique via un point de liaison (5.3.1, 5.3.2, 5.3.3).

11. Dispositif selon l'une quelconque des revendications précédentes, **caractérisé en ce que** deux étais (5.1.1, 5.1.2) disposés côte à côte dans la direction périphérique sont reliés aux étais (5.1.1, 5.1.2) directement connexes à lui dans le plan axial via un point de liaison (5.3.1, 5.3.2).

12. Dispositif selon l'une quelconque des revendications précédentes, **caractérisé en ce qu'**en cas de partie d'écartement (5) écartée, quatre étais (5.1.1, 5.1.2 et/ou 5.1.2, 5.1.3) ceignent un espace intermédiaire (5.2.1, 5.2.2) en forme de losange.

13. Dispositif selon l'une quelconque des revendications précédentes, **caractérisé en ce que** dans la configuration écartée, les étais (5.1.1, 5.1.2) successifs entourant un espace intermédiaire (5.2.1) en forme de losange ont une longueur différente, les étais (5.1.1, 5.1.2) successifs de longueur différente étant notamment réalisés dans la région proximale de la partie d'écartement (5).

14. Dispositif selon l'une quelconque des revendications précédentes, **caractérisé en ce que** dans la configuration écartée, les étais (5.1.2) successifs délimitant dans le plan axial un espace intermédiaire (5.2.2) en forme de losange présentent une longueur identique, les étais (5.1.2) successifs comportant notamment une longueur identique dans la zone distale de la partie d'écartement (5).

15. Dispositif selon l'une quelconque des revendications précédentes, **caractérisé en ce que** les extrémités distales libres des étais (5.1.2) sont arrondies.

16. Dispositif selon l'une quelconque des revendications précédentes, **caractérisé en ce que** les tubes extérieur et intérieur (2.2, 3.2) comportent au niveau de leurs extrémités proximales des parties de poignée (2.1, 3.1) interagissant entre elles, ces parties formant une poignée (4) commune dans la configuration écartée de la partie d'écartement, une section (3.1.3) distale de la partie de poignée (3.1) de la partie intérieure (3) comportant notamment un filetage extérieur interagissant avec une tige (2.1.3) de la partie de poignée (2.1) du tube extérieur (2) s'imbriquant dans ladite partie intérieure et saillant vers l'intérieur.

17. Dispositif selon l'une quelconque des revendications précédentes, **caractérisé en ce que** le tube extérieur (2.2) est relié par complémentarité de formes à une première partie de poignée (2.1) d'une poignée (4) et/ou que le tube intérieur (3) est relié par complémentarité de formes à une deuxième partie de poignée (3.1) d'une poignée, le tube extérieur (2.2) et/ou le tube intérieur (3.2) comportant notamment respectivement au niveau de son extrémité proximale des becs (2.2.2, 3.2.2) orientés vers l'extérieur avec des détourés arrière s'imbriquant avec les détourés arrière (2.1.2, 3.1.2) réalisés sur le côté intérieur de la partie de poignée respective.
